# EUROPEAN PATENT APPLICATION

(11) **EP 2 153 808 A1**
(43) Date of publication of application: **17.02.2010**
(21) Application number: 08162062.7
(22) Date of filing: 08.08.2008
(51) Int. Cl.: A61F 13/15, C08B 11/14, C08B 15/10

(54) **Absorbent product comprising a cationic modified guar gum**

(71) Applicant: The Procter and Gamble Company, Cincinnati, Ohio 45202 (US)
(72) Inventor: Carlucci, Giovanni, 66100, Chieti (IT); Gagliardini, Alessandro, 65010, Moscufo (Pescara) (IT)
(74) Representative: Veronese, Pancrazio

(57) **Abstract**

Absorbent products for feminine protection, for example sanitary pads, pantiliners or tampons, comprising a cationic modified guar gum. The guar gum is modified by a cationizing agent comprising an ammonium group, wherein the degree of substitution of the cationizing agent is from about 0.070 to less than 0.30. In one aspect of the invention, the cationic modified guar gum is substantially water soluble.

## Description

### FIELD OF THE INVENTION

The invention relates to absorbent products for feminine protection, for example sanitary pads, pantiliners or tampons, comprising a cationic modified guar gum.

### BACKGROUND OF THE INVENTION

Most commercially available disposable absorbent products like sanitary napkins and diapers comprise synthetic superabsorbent polymers (SAP), typically polyacrylates, to deliver body fluid absorption and retention characteristics. Although such synthetic absorbent materials exhibit outstanding absorption capacity towards de-ionized water, their absorption capacity towards electrolytes/salts-containing solutions like menses is lower. It is assumed that the presence of electrolytes, proteins and cells (mainly red cells in menses) interfere with the swelling process of the absorbing gelling materials (see for ref. P.K. Chatterjee, B. S. Gupta, "Absorbent Technology" Elsevier 2002; pages 455-457).

Whereas synthetic superabsorbent polymers have been found to work very well to absorb simple fluids like urine, their performance is disappointing in feminine care applications where at least part of the fluid to be absorbed is menstrual fluid. This can lead to the failure of the feminine care product to efficiently absorb the menstrual fluid and eventually leakage and soiling of the user's garments.

US 2004/0122390A1 discloses low evaporative absorbent products. The low evaporative absorbent products comprise a treatment agent in the absorbent core of the absorbent product which, upon activation, coats swollen superabsorbent products present in the absorbent core, among which gums are disclosed, to reduce evaporation therefrom. Several suitable treatment agents are disclosed.

US 5,780,616 discloses cationic polysaccharides having superabsorbent characteristics. The polysaccharides are substituted by quaternary ammonium groups, having a relatively high degree of substitution of at least 0.5. The polysaccharide is preferably cellulose. The polysaccharides are cross-linked to a sufficient extent that they remain insoluble in water.

US 6,887,564, to Procter & Gamble, discloses disposable absorbent products comprising chitosan material and an anionic absorbent gelling material. However, the high cost of chitosan materials has prevented until now their commercial uses.

US 5,532,350 discloses crosslinked, polysaccharides useful as absorbent material, among which guar gum and guar derivatives are typically used. The polysaccharides are water insoluble

US 5,801,116 discloses crosslinked or not crosslinked polysaccharides, particularly guar polymers, such as for example guar gum, used as absorbent materials. The polysaccharides are typically water insoluble, or only slightly water soluble, having less than 50% of the polysaccharide which dissolves in water.

From the above considerations there is the need for a material having a high ability to immobilize menses at an affordable price.

It has now surprisingly been found that certain cationic modified guar gums can deliver performance comparable to chitosan derivatives in absorbent products for feminine protection. The modified guar gums of the invention may be more water soluble than previously suggested. The modified guar gums of the invention may be synthesized typically without crosslinking them, or also by using relatively lower level of cross-linking agent than previously suggested. It was also found that a relatively low degree of substitution for the quaternary ammonium groups was adequate for feminine protection applications.

Cationic modified guar gums can have the further advantage to come from a raw material (guar gum) largely available and potentially cheaper compared for example to chitosan salts.

The cationic modified guar gums of the invention perform particularly well in presence of proteinaceous fluids such as menses and deliver fluid-handling benefits, and may have better ability to increase the viscosity of blood-based fluids than other polysaccharide derivatives already described. Without wishing to be bound by theory, it is believed that the cationic modified guar gums of the invention have a unique combination of characteristics that make them optimized towards menses immobilization.

### SUMMARY OF THE INVENTION

The invention is for an absorbent product for feminine protection comprising a cationic modified guar gum, wherein said cationic modified guar gum comprises guar gum modified by a cationizing agent comprising an ammonium group, wherein the degree of substitution of the cationizing agent is from about 0.070 to less than 0.30. In one aspect of the invention, the cationic modified guar gum is substantially water soluble.

In another aspect, the invention is also for a method for making an absorbent product for feminine protection, comprising the steps of:
- reacting guar gum with a cationizing agent comprising an ammonium group, so that a cationic modified guar gum having a degree of substitution of the cationizing agent of from about 0.070 to less than 0.30 is obtained;
- optionally reacting in the same or in a separate step the guar gum with a cross-linking agent at a concentration of from about 0 ppm to about 1000 ppm of cross-linking agent by weight of guar gum;
- applying the cationic modified guar gum obtained by the preceding steps to a component of an absorbent product for feminine protection, for example an absorbent core;
- making an absorbent product for feminine protection using said component.

### DETAILED DESCRIPTION OF THE INVENTION

The term "absorbent product for feminine protection" refers to products normally used by women for absorbing menses as well as adult light to moderate incontinence products. These products are usually disposable, i.e. are discarded after usage. Usual absorbent products for feminine protection include menses absorbing products such as sanitary napkins, pantiliners, tampons, and interlabial pads but do not include infant diapers.

The term "cationic modified guar gum" as used herein refers to the product of the reaction between guar gum and a suitable cationizing agent. Usually, cationic modified guar gums may have a net positive charge in aqueous solutions at a pH range from 3 to 10, in particular between pH of 5 and 9.

The absorbent products for feminine protection of the present invention comprise a cationic modified guar gum. The source of guar gum before cationic modification is typically the guar bean.

Guar gum, also known as guar flour, comprises high molecular weight polysaccharides composed of galactomannans. The water soluble fraction of guar gum is called guaran and typically consists of linear chains of (1→6)-β-D-mannopyranosyl units (D-mannose) with α-D-galactopyranosyl units (D-galactose) attached by (1→6) linkages. Ratio of D-galactose to D-mannose is 1:2.

Various methods for providing guar gums with cationic functionalityare known in the art, for example as disclosed in US 2008/0112907, which however describes water dispersible polygalactomannan polymers. Various methods for cross-linking guar gums with and without cationic modification of the guar gums are also known, see for example US 5,532,350 and US 5,801,116. The cationic modified guar gums used in the present invention can be easily made by a skilled person using these known chemical reactions. The cationic agents used in the fabrication of the modified guar gums of the invention can typically comprise an ammonium group.

Suitable cationic agents comprising an ammonium group can include for example those listed in US 5,780,616 col.4 line 5 to col. 5 line 15, and in US 2008/0112907. In particular the following examples:
- 2,3-epoxypropyl-N,N,N-trimethylammonium chloride (commercially available from Degussa A. G. as a 70% aqueous solution under the name QUAB 151 or as the pure compound in solid form from Fluka under product code 50045) having for structural formula: 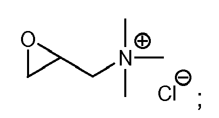
- 3-chloro-2-hydroxypropyl-N,N,N-trimethylammonium chloride (CAS # 3327-22-8, commercially available from Degussa A. G. as a 65% aqueous solution under the name of QUAB 188), having the structural formula:

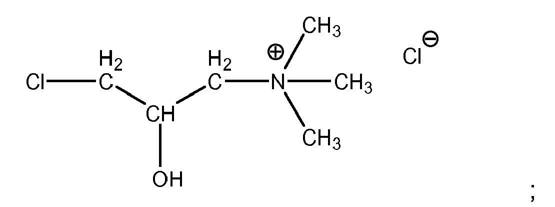

The extent of the cationization of a polysaccharide by a cationizing agent may be expressed (as is common in the art, see for example US 5,780,616, GB 1576475, US 7,135,451B1), by using the degree of substitution of the reactive groups of the polysaccharide by the cationizing agent (herein referred to as the "degree of substitution of the cationizing agent"). The degree of substitution of the cationizing agent can be measured by any conventional methods, for example the method disclosed in WO92/19652, or the method disclosed in US 7,135,451 col. 3, or for example a method based on elemental analysis by measuring the amount of nitrogen bound to the modified guar gum (when the cationizing agent is based on an ammonium group), such as for example the Kjeldahl method. All these methods are well known in the art.

The inventors have found that modified guar gums with a low degree of substitution of cationizing agent may provide better benefits for immobilizing menses. The cationic modified guar gums of the invention can have a degree of substitution of cationizing agent in the range of from about 0.070 to less than 0.30, in particular from about 0.10 to about 0.20.

Whereas at least some of the prior art documents teach using insolubilized modified guar gums, the inventors have found that the modified guar gums of the invention can be substantially water soluble and provide the desired properties. Thus in one aspect of the invention, the modified guar gums of the invention are substantially water-soluble. By "substantially water-soluble", we mean that they contain less than 50% (by weight) of water-insoluble carbohydrate, e.g. as determined by the test disclosed in GB 1,576,475. More specifically, the modified guar gum of the invention may contain less than 30%, or less than 20% or even less than 10% of insoluble carbohydrate. In one embodiment, the modified guar gum of the invention may be entirely water soluble. The solubility data can be measured as follows:

The modified guar gum (1 g) is slurried in distilled water (100 ml) at room temperature (21 °C) with stirring for 15 minutes. The slurry is allowed to stand for 8 hours before filtering. The dissolved carbohydrate in the filtrate is measured by the known colorimetric method employing the use of the phenol/sulphuric acid test for soluble carbohydrate. In these determinations to 1 ml of the sample of the test solution is added 1 ml of phenol solution (5% w/v) followed by 5 ml of concentrated sulphuric acid and the liquids mixed by hand shaking for one minute. After leaving to cool for an hour the concentration of the soluble carbohydrate is determined using a ultraviolet spectrophotometer (e.g. Unicam SP 800 or similar) from the absorbency at the peak at 483 nm by reference to a glucose standard.

The solubility of modified guar gums is normally driven by the amount of cross-linking agent used. Whilst at least some of the prior art (for example US 5,532,350 and US 5,801,116) teaches to use high degree of cross-linking in order to render the modified guar gums substantially water insoluble, the inventors have found that low levels of cross-linking can be beneficial for feminine care applications. In a typical embodiment of the present invention, the modified guar gums can have no crosslinking. However, the modified guar gums of the invention may still be advantageously slightly cross-linked, in particular in order to increase their processability and the recovery of the modified guar gum during the synthesis, but the levels of cross-linking may be advantageously lower than disclosed in the prior art.

The level of cross-linking of a modified guar gum and hence the solubility of the modified guar gum can be controlled by the skilled person during the synthesis, in particular the concentration of cross-linking agents in the reaction mixture can be varied to obtain the desired amount of cross-linking.

In another aspect of the invention, it was found that a concentration of cross-linking agent of from about 0 ppm to about 1000 ppm (parts per million) in the reaction mixture may be advantageous to obtain the desired amount of cross-linking. More particular ranges are from about 50 ppm to 700 ppm, and from about 100 ppm to 700 ppm. By "ppm" we mean the relative amount of the cross-linking agent expressed in weight units per weight of the guar gum material to be cross-linked expressed in parts per million.

The amount of cross-linking can also be expressed by reference to the degree of substitution of the modified guar gum by the cross-linking agents (herein referred to as "degree of substitution of the cross-linking agent"), which may advantageously be less than 0.0010, for example from about 0.00001 to about 0.00025, or from about 0.00003 to about 0.000200. The degree of substitution of the cross-linking agent is sometimes used in the literature (see GB 1,576,475 and US 3,622,562 for example).

Crosslinking of guar gum can be made according to various methods known in the art, for example as disclosed in US 5,532,350, US 5,801,116 and in US 2008/0112907.

Typically crosslinking can be achieved by means of suitable crosslinking agents added to the guar gum. Typical crosslinking agents can include aluminium, titanium or zirconium compounds, such as for example aluminium, titanium or zirconium salts. Copper, iron, lead, calcium and sodium salts can also be used as crosslinking agents. Other typical crosslinking agents may include borate materials such as borax. In an embodiment of the present invention the crosslinking agent can be glyoxal.

As indicated above, methods for making the cationic modified guar gums of the present invention with or without cross-linking are well known in the art.

The cationic modified guar gums can be applied to the absorbent product in a number of ways. For example, a water or solvent based solution of the cationic modified guar gum may be applied. It is also possible to apply the cationic modified guar gum in a dry powder form.

According to an embodiment of the present invention, the cationic modified guar gum is comprised in an absorbent article in particulate form having an average particle size of less than 100 µ, or between 1 µ and 70 µ, or between 5 µ and 50 µ, or also between 10 µ and 30 µ. It has been discovered that relatively low average particle size of the cationic modified guar gum can be advantageous for menses immobilization in an absorbent article. The average particle size can be evaluated with any of the methods known in the art; for relatively low particle sizes, such as those disclosed above, methods based on light scattering technique can be advantageously used. As it is known in the art, the average particle size, also called median particle size, is typically evaluated from the particle size distribution, obtained with suitable known means, of a particulate material, and is typically taken as the particle size which divides in two said distribution, i.e. such that half by weight of the particulate material is coarser than said average particle size, and half by weight is finer. The average, or median, particle size of the cationic modified guar gum in particulate form can be measured for example by means of a laser diffraction particle size analyzer LS 13 320 Series available from Beckman Coulter, or of any equivalent apparatus.

In general, the measurement of the average particle size shall be conducted in a controlled environment, typically at 23±2°C and 50±10% RH, on a dry sample material. The sample material shall be dried in an oven for three hours at 60°C, then kept in a closed container and allowed to equilibrate to the ambient laboratory temperature. Care shall be taken to test the sample material quickly, i.e. typically in no more than 45 minutes after removal from the closed container in order to minimize moisture gain in the controlled laboratory environment.

The cationic modified guar gum may be applied to one component of the absorbent product before the component is used in the making of the product. For example, for absorbent products such as sanitary napkins or pantiliners, which normally comprise a liquid pervious topsheet, a backsheet and an absorbent core intermediate the backsheet and the topsheet, the cationic modified guar gum may be applied to any of these components. Advantageously, the cationic modified guar gum may be applied to the core. In that case, the cationic modified guar gum may be applied on a portion or the totality of one or both of the surfaces of the absorbent core, such as the body facing surface of the core, the garment facing surface of the core or both surfaces of the core. The cationic modified guar gum may also be applied on a central portion of one of the surface core, or as stripes along two sides of the core. It may also be envisaged to apply the cationic modified guar gum within one of the component of the absorbent product, for example for a core which is made as a laminate of several layers, the surface of one of these layers which is not an external surface of the core may be applied with the cationic modified guar gum. The cationic modified guar gum may also be applied to other components of the products if present such as backsheet or topsheet.

According to an embodiment of the present invention, the cationic modified guar gum can be applied to the absorbent product, namely to any of the components thereof, for example on at least a portion of at least one of the surfaces of the absorbent core, in a concentration of from 0.5 g/m² to 500 g/m², preferably from 1 g/m² to 50 g/m², by weight of the cationic modified guar gum per square meter of the zone of application.

When spraying an aqueous solution of modified cationic guar gum, the aqueous solution may exemplarily comprise cationic guar gum at a concentration of from about 3% to about 6% by weight of the solution. For solvent based solution, it is envisaged that higher concentration may be used, for example from about 6% to about 60% by weight of the solution. It is envisaged that the cationic modified guar gum may be advantageously applied to an individual component of the absorbent product before this component is assembled with the other components forming the product, or may be applied to the finished product. The invention is thus also for a method for making an absorbent product for feminine protection, comprising the steps of:
- reacting guar gum with a cationizing agent comprising an ammonium group, so that a cationic modified guar gum having a degree of substitution of the cationizing agent of from about 0.070 to less than 0.30 is obtained;
- optionally reacting in the same or in a separate step said guar gum with a cross-linking agent at a concentration of from about 0 ppm to about 1000 ppm of cross-linking agent by weight of guar gum;
- applying the cationic modified guar gum obtained by the preceding steps to a component of an absorbent product for feminine protection;
- using said component to make an absorbent product for feminine protection.

The cationic modified guar gum may be applied to the component before or after the component is used to make the absorbent product. Advantageously, the step of applying the cationic modified guar gum may precede the step of using the component to make the absorbent product for feminine protection. For example, the cationic modified guar gum may be applied to the absorbent core before the core is used to make the absorbent product.

If an absorbent core is used in the product of the invention, any types of absorbent core may be used. Standard cores usually comprise a fluff matrix of cellulose pulp, or a mixture of cellulose pulp with synthetic fibers. The core may also comprise classic synthetic superabsorbent materials such as polyacrylate based gelling material. Thin cores such as those disclosed for example in EP1447067 may also be used.

The absorbent products of the invention may further comprise an anionic absorbent gelling material, for example in the absorbent core.

### EXPERIMENTALS

### Rheological analysis

One exemplary way to compare the effectiveness of the modified guar gums in immobilizing menses may be done by the rheological analysis, for example as indicated below.

Samples preparation: a 5% w/w water solution of the cationic modified guar gum is prepared weighing 0.5 g of cationic guar gum powder into a Petri dish of 12x12 cm. Then 4.50 g of distilled water are added and the mixture is stirred until a homogeneous gel is obtained into the Petri dish.

After gelling, the Petri dish is placed into a ventilated oven with temperature set at 45° C. for 8 hrs until complete dryness.

After drying into the Petri dish, a layer of dried cationic modified guar gum powder will be present. The Petri dish is removed from the oven and kept 1 hour to equilibrate at room temperature.

To the dried cationic polymer layer are slowly added 4.50 g of AMF (Artificial Menstrual Fluid, described below), wetting the entire surface. After the addition, the material is further mixed with a spatula for 5 min until a homogeneous gel is obtained.

Evaluation of rheological properties: rheology parameter (G') has been measured using a Stress Tech HR rheometer supplied by Reologica Instruments Inc 231 Crosswicks Road Bordentown, NJ 08505 USA. This instrument was operated using a parallel plate geometry of 40 mm with a gap of 2 mm, at 40 Hz and 37°C. Software used was RheoExplorer version 5.0.40.38.

G' is the elastic modulus and the values measured at 40 Hz are considered representative of the ability of the cationic modified guar gum to thicken artificial menses fluid.

It is believed that the higher the value of G' the better the modified guar gum can immobilize menses in an absorbent product.

### Preparation of AMF (Artificial Menstrual Fluid)

Artificial Menstrual Fluid (AMF) is based on modified sheep's blood that has been modified to ensure it closely resembles human menstrual fluid in viscosity, electrical conductivity, surface tension and appearance.

### Reagents:

Difibrinated sheep's blood is available from Unipath S.p.A. (Garbagnate Milanese/Italy).
Lactic acid from J. T. Baker Holland Reagent Grade (85-95 w/w).
Potassium Hydroxyde (KOH) from Sigma Chemical Co. USA Reagent grade.
Phosphate buffer saline tablets from Sigma Chemical Co. USA, Reagent grade.
Sodium Chloride from Sigma Chemical Co. USA, Reagent grade.
Gastric Mucine from Sigma Chemical Co. USA, Type III (CAS 84082-64-4).
Distilled water.

Step 1: Prepare a 9 +/- 1% Lactic Acid solution by dissolution of lactic acid powder and distilled water.
Step 2: Prepare a 10% Potassium Hydroxyde (KOH) solution by dissolving KOH powder into distilled water.
Step 3: Prepare a Phosphate buffer solution buffered to pH=7.2 by dissolving tablets as directed into 1 L distilled water.
Step 4: Prepare and slowly heat to 45 +/- 5° C. a solution of the following composition: 460 +/-5 ml of phosphate buffer solution. 7.5 +/- 0.5 ml of KOH solution.
Step 5: Prepare a Mucous Solution by slowly dissolution (with constant stirring) of approximately 30 grams of gastric mucine in the pre-heated (45 +/-5°C.) solution prepared in step 4. Once dissolved the solution temperature should be increased to between 50-80°C. and the mixture covered for approximately 15 min. Turn the heat down to maintain a relatively constant temperature between 40 and 50°C. and continue to stir for a period of 2.5 hrs.
Step 6: Remove the solution from the hot plate and allow the solution (from step 5) to now cool to less than 40° C. Add 2.0 ml of the 10% lactic acid solution and mix thoroughly for 2 min.
Step 7: Place the solution in an autoclave and heat to a temperature of 121°C. for 15 min.
Step 8: Allow the solution to cool to room temperature and dilute 1 to 1 with the defibrinated sheep's blood.

Following AMF preparation, its viscosity, pH and conductivity are measured to ensure the blood characteristics lie in a range close to that of normal menstrual blood (see reference H. J. Bussing "Zur Biochemie des Menstrualblutes" Zbl Gynaec, 179,456 (1957)). The viscosity should lie in the range of 7 to 8 (units cStK). The pH should lie in the range of 6.9 to 7.5 and the conductivity in the range 10.5 to 13 (units mmho). If the viscosity is not within the range specified above it should not be used and a new batch of AMF needs to be prepared. The AMF solution must be constantly mixed to ensure the components do not separate prior to usage. The solution should be used only within 4 hours of preparation.

## Claims

1. An absorbent product for feminine protection comprising a cationic modified guar gum, wherein said cationic modified guar gum comprises guar gum modified by a cationizing agent comprising an ammonium group, **characterized in that** the degree of substitution of the cationizing agent is from 0.070 to less than 0.30, preferably from 0.10 to 0.20, and **in that** the cationic modified guar gum is substantially water soluble.

2. A product according to any preceding claim, wherein the cationic modified guar gum is cross-linked by a cross-linking agent.

3. An absorbent product according to claim 2, wherein the cross-linking agent is reacted with the guar gum in concentration of from 0 ppm to 1000 ppm, preferably from 50 ppm to 700 ppm, more preferably of from 100 ppm to 500 ppm,of cross-linking agent by weight of the guar gum.

4. An absorbent product for feminine protection comprising a cationic modified guar gum, wherein said guar gum is modified by a cationizing agent comprising an ammonium group and wherein the cationic modified guar gum is cross-linked by a cross-linking agent, **characterized in that** the degree of substitution of the cationizing agent is from 0.070 to less than 0.30 and that the cross-linking agent has been reacted with the guar gum in concentration of from 0 ppm to 1000 ppm of cross-linking agent by weight of guar gum.

5. An absorbent product according to any of claims 2 to 4 wherein the cross-linking agent is glyoxal.

6. A product according to any preceding claim, wherein the cationic modified guar gum is in particulate form, and has an average particle size of less than 100 µ, preferably between 1 µ and 70 µ, more preferably between 5 µ and 50 µ, most preferably between 10 µ and 30 µ.

7. An absorbent product according to any of the preceding claims wherein said product is selected from the group consisting of a sanitary napkin, pantiliner, tampon and interlabial pad.

8. An absorbent product according to any of the preceding claims wherein said product comprises a liquid pervious topsheet, a backsheet and an absorbent core intermediate said backsheet and said topsheet.

9. An absorbent product according to claim 8 wherein the cationic modified guar gum is applied on at least a portion of at least one of the surfaces of the absorbent core.

10. An absorbent product according to claim 9 wherein the cationic modified guar gum is applied to the surface in a concentration of from 0.5 g/m² to 500 g/m², preferably from 1 g/m² to 50 g/m², by weight of the cationic modified guar gum per square meter of the zone of application.

11. An absorbent product according to any of the preceding claims further comprising an anionic absorbent gelling material.

12. A method for making an absorbent product for feminine protection, comprising the steps of:
- reacting guar gum with a cationizing agent comprising an ammonium group, so that a cationic modified guar gum having a degree of substitution of the cationizing agent of from 0.070 to less than 0.30 is obtained;
- optionally reacting in the same or in a separate step said guar gum with a cross-linking agent at a concentration of from 0 ppm to 1000 ppm of cross-linking agent by weight of guar gum;
- applying the cationic modified guar gum obtained by the preceding steps to a component of an absorbent product for feminine protection;
- using said component to make an absorbent product for feminine protection.

13. A method according to claim 12, wherein said cationic modified guar gum is applied in particulate form with an average particle size of less than 100 µ, preferably between 1 µ and70 µ, more preferably between 5 µ and 50 µ, most preferably between 10 µ and 30 µ.

14. A method according to claim 12 or 13 wherein said component on which the cationic modified guar gum is applied is an absorbent core.

15. An method according to any claim 12 to 14 wherein the cationic modified guar gum is applied in a concentration of from 0.5 g/m² to 500 g/m², preferably from 1 g/m² to 50 g/m², by weight of the cationic modified guar gum per square meter of the zone of application.
